(19) Europäisches Patentamt / European Patent Office / Office européen des brevets

(11) **EP 3 270 969 B1**

(12) **EUROPEAN PATENT SPECIFICATION**

(45) Date of publication and mention of the grant of the patent:
**23.01.2019 Bulletin 2019/04**

(51) Int Cl.:
*A61K 47/38* (2006.01)     *A61K 9/16* (2006.01)
*C08B 11/20* (2006.01)     *C08B 13/00* (2006.01)

(21) Application number: **16711735.7**

(22) Date of filing: **08.03.2016**

(86) International application number:
**PCT/US2016/021322**

(87) International publication number:
**WO 2016/148975 (22.09.2016 Gazette 2016/38)**

(54) **A PROCESS FOR FRACTIONATING AN ESTERIFIED CELLULOSE ETHER**

VERFAHREN ZUR FRAKTIONIERUNG EINES VERESTERTEN CELLULOSEETHERS

PROCÉDÉ DE FRACTIONNEMENT D'UN ÉTHER DE CELLULOSE ESTÉRIFIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2015   US 201562133505 P**

(43) Date of publication of application:
**24.01.2018   Bulletin 2018/04**

(73) Proprietor: **Dow Global Technologies LLC
Midland, MI 48674 (US)**

(72) Inventors:
• **PETERMANN, Oliver
29699 Bomlitz (DE)**
• **KNARR, Matthias
29699 Bomlitz (DE)**
• **BRACKHAGEN, Meinolf
29699 Bomlitz (DE)**
• **SPREHE, Matthias
29699 Bomlitz (DE)**

(74) Representative: **f & e patent
Braunsberger Feld 29
51429 Bergisch Gladbach (DE)**

(56) References cited:
**WO-A1-2014/031419     WO-A1-2014/031422
WO-A1-2014/031447     WO-A1-2014/137778
WO-A1-2014/137779     WO-A1-2014/137789**

**Description**

FIELD

This invention concerns a process for fractionating an esterified cellulose ether, a novel process for preparing an esterified cellulose ether and esterified cellulose ethers obtainable from such processes.

INTRODUCTION

Esters of cellulose ethers, their uses and processes for preparing them are generally known in the art. When the esterified cellulose ethers comprise ester groups which carry carboxylic groups, the solubility of the esterified cellulose ethers in aqueous liquids is typically dependent on the pH. For example, the solubility of hydroxypropyl methyl cellulose acetate succinate (HPMCAS) in aqueous liquids is pH-dependent due to the presence of succinate groups, also called succinyl groups or succinoyl groups. HPMCAS is known as enteric polymer for pharmaceutical dosage forms. In the acidic environment of the stomach HPMCAS is protonated and therefore insoluble. HPMCAS undergoes deprotonation and becomes soluble in the small intestine, which is an environment of higher pH. The pH-dependent solubility is dependent on the degree of substitution of acidic functional groups. The dissolution time of various types of HPMCAS dependent on pH and on the degree of neutralization of HPMCAS is discussed in detail in McGinity, James W. Aqueous Polymeric Coatings for Pharmaceutical Dosage Forms, New York: M. Dekker, 1989, pages 105 - 113. The above-mentioned article *Aqueous Polymeric Coatings for Pharmaceutical Dosage Forms* illustrates in Fig. 16 on p. 112 the dissolution time of several grades of HPMCAS, which have different degrees of substitution with succinoyl, acetyl and methoxyl groups, in pure water and in 0.1 N NaCl depending on the degree of neutralization of the HPMCAS. Depending on the HPMCAS and the presence or absence of NaCl, HPMCAS is soluble when it has a degree of neutralization between about 0.55 and 1. Below a degree of neutralization of about 0.55, all HPMCAS grades are insoluble in pure water and in 0.1 N NaCl.
Dosage forms coated with esterified cellulose ethers such as HPMCAS protect the drug from inactivation or degradation in the acidic environment of the stomach or prevent irritation of the stomach by the drug but release the drug in the small intestine. US Patent No. 4,365,060 discloses enterosoluble capsules. U.S. Patent No. 4,226,981 discloses a process for preparing mixed esters of cellulose ethers, such as HPMCAS.
It is also known in the prior art, e.g. as published in International Patent Application WO 2005/115330, that HPMCAS is also useful to increase the bioavailability of poorly water-soluble drugs. This is of great importance as nearly 70% of new drug candidates are low water soluble compounds. As a general rule, poorly water soluble drugs possess low bioavailability. The HPMCAS is aimed at reducing the crystallinity of the drug, thereby minimizing the activation energy necessary for the dissolution of the drug, as well as establishing hydrophilic conditions around the drug molecules, thereby improving the solubility of the drug itself to increase its bioavailability, i.e., its *in vivo* absorption by an individual upon ingestion.
The interaction of esterified cellulose ethers with drugs to increase their bioavailability depends on various factors, such as the degree of substitution with ether and ester groups. As the molecular weight of polymers is known to have a large influence on their properties, the skilled artisans have good reasons to believe that this interaction also depends on the molecular weight of the esterified cellulose ethers. Hence, it is desirable to find an efficient way of controlling and/or adjusting the molecular weights, such as the weight average molecular weight $M_w$, of esterified cellulose ethers. A process for preparing esters of a cellulose ether having different weight average molecular weights is disclosed in WO 2014/031447.

SUMMARY

Surprisingly, an efficient and simple process for fractionating esterified cellulose ethers has been found. The general term "polymer fractionation" means the targeted manipulation of the molecular weight distribution of a polymer by removing short and/or long chain material.
One aspect of the present invention is a process for fractionating an esterified cellulose ether comprising groups of the formula -C(O)-R-COOH, wherein R is a divalent hydrocarbon group, which process comprises the steps of

a) blending the esterified cellulose ether comprising groups of the formula -C(O)-R-COOH with an aqueous liquid and setting the temperature of the resulting blend to less than 10 °C to dissolve a portion of the esterified cellulose ether in the aqueous liquid,
b) separating the non-dissolved portion of the esterified cellulose ether from the remainder of the blend, and
c) recovering or disposing of the esterified cellulose ether that is dissolved in the aqueous liquid.

Another aspect of the present invention is a process for preparing an esterified cellulose ether comprising groups of the formula -C(O)-R-COOH, wherein R is a divalent hydrocarbon group, which process comprises the steps of

a) reacting a cellulose ether with a dicarboxylic acid anhydride or with a combination of a dicarboxylic acid anhydride and an aliphatic monocarboxylic acid anhydride in the presence of an aliphatic carboxylic acid to produce a reaction product mixture comprising an esterified cellulose ether comprising groups of the formula -C(O)-R-COOH, precipitating the esterified cellulose ether from the reaction product mixture, blending the precipitated esterified cellulose ether with an aqueous liquid and setting the temperature of the resulting blend to less than 10 °C to dissolve a portion of the esterified cellulose ether in the aqueous liquid,
b) separating the non-dissolved portion of the esterified cellulose ether from the remainder of the blend, and
c) recovering or disposing of the esterified cellulose ether that is dissolved in the aqueous liquid.

Another aspect of the present invention is an esterified cellulose ether obtainable from step b) of the processes of the present invention.

Yet another aspect of the present invention is an esterified cellulose ether obtainable from step c) of the processes of the present invention.

DESCRIPTION OF EMBODIMENTS

Surprisingly, it has been found that a lower molecular portion of an esterified cellulose ether comprising groups of the formula -C(O)-R-COOH is dissolved in an aqueous liquid when the esterified cellulose ether is blended with the aqueous liquid as defined further below and the temperature of the resulting blend is set to a temperature of less than 10 °C, preferably less than 8°C, more preferably less than 5 °C, and particularly 3 °C or less. The higher molecular portion of the esterified cellulose ether remains un-dissolved, even at a temperature of less than 10 °C. When the temperature of the blend has a temperature of 10 °C or more, such partial dissolution is not observed. Particularly at room temperature known esterified cellulose ethers comprising groups of the formula -C(O)-R-COOH do not dissolve in water to a noticeable degree when the degree of neutralization of the groups -C(O)-R-COOH of the esterified cellulose ethers is less than 0.55.

The esterified cellulose ether has a cellulose backbone having β-1,4 glycosidically bound D-glucopyranose repeating units, designated as anhydroglucose units in the context of this invention. The cellulose ether used as a starting material in the process of the present invention preferably is an alkyl cellulose, hydroxyalkyl cellulose or hydroxyalkyl alkylcellulose. This means that in the cellulose ether utilized in the process of the present invention, at least a part of the hydroxyl groups of the cellulose backbone at the 2-, 3- and 6-positions of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred. Illustrative of the above-defined cellulose ethers are alkylcelluloses, such as methylcellulose, ethylcellulose, and propylcellulose; hydroxyalkylcelluloses, such as hydroxyethylcellulose, hydroxypropylcellulose, and hydroxybutylcellulose; and hydroxyalkyl alkylcelluloses, such as hydroxyethyl methylcellulose, hydroxymethyl ethylcellulose, ethyl hydroxyethylcellulose, hydroxypropyl methylcellulose, hydroxypropyl ethylcellulose, hydroxybutyl methylcellulose, and hydroxybutyl ethylcellulose; and those having two or more hydroxyalkyl groups, such as hydroxyethylhydroxypropyl methylcellulose. Most preferably, the cellulose ether is a hydroxypropyl methylcellulose.

The degree of the substitution of hydroxyl groups at the 2-, 3- and 6-positions of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl). The cellulose ether utilized in the process of the invention generally has a molar substitution of hydroxyalkoxyl groups in the range 0.05 to 1.00, preferably 0.08 to 0.90, more preferably 0.12 to 0.70, most preferably 0.15 to 0.60, and particularly 0.20 to 0.40.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The esterified cellulose ethers preferably have a DS(alkoxyl) in the range of 1.0 to 2.5, more preferably from 1.1 to 2.4 , most preferably from 1.2 to 2.2 and particularly from 1.6 to 2.05.

Most preferably the esterified cellulose ether is an esterified hydroxypropyl methylcellulose having a DS(methoxyl) within the ranges indicated above for DS(alkoxyl) and an MS(hydroxypropoxyl) within the ranges indicated above for MS(hydroxyalkoxyl). The esterified cellulose ether comprises groups of the formula -C(O)-R-COOH, wherein R is a divalent hydrocarbon group, such as -C(O)-CH$_2$-CH$_2$-COOH, and optionally aliphatic monovalent acyl groups, such as acetyl, propionyl, or butyryl, such as n-butyryl or i-butyryl. Specific examples of esterified cellulose ethers are hydroxypropyl methylcellulose acetate succinate (HPMCAS), hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS), or methyl cellulose acetate succinate (MCAS). Hydroxypropyl methylcellulose acetate succinate (HPMCAS) is the most preferred esterified cellulose ether.

The esterified cellulose ether generally has a degree of substitution of groups of formula -C(O)-R-COOH, such as succinoyl, of at least 0.01, preferably at least 0.05, and most preferably at least 0.10. The esterified cellulose ethers generally have a degree of substitution of groups of formula -C(O)-R-COOH of up to 0.90, preferably up to 0.80, and more preferably up to 0.50. The esterified cellulose ethers generally have a degree of substitution of aliphatic monovalent acyl groups, such as acetyl, propionyl, or butyryl groups, of 0 or at least 0.05, preferably at least 0.10, and more preferably at least 0.25. The esterified cellulose ethers generally have a degree of substitution of aliphatic monovalent acyl groups of up to 0.95, preferably up to 0.80, and more preferably up to 0.70. The total degree of ester substitution is generally at least 0.05, preferably at least 0.10, and more preferably at least 0.20. The total degree of ester substitution is generally not more than 1.0, preferably not more than 0.90, and more preferably not more than 0.80.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl and other ester groups.

The content of ether groups in the esterified cellulose ether is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

$$
\begin{aligned}
\% \text{ cellulose backbone} \\
= 100 - &\left( \%\text{MeO} * \frac{M(OCH_3) - M(OH)}{M(OCH_3)} \right) \\
- &\left( \%\text{HPO} * \frac{M(OCH_2CH(OH)CH_3) - M(OH)}{M(OCH_2CH(OH)CH_3)} \right) \\
- &\left( \%\text{Acetyl} * \frac{M(COCH_3) - M(H)}{M(COCH_3)} \right) \\
- &\left( \%\text{Succinoyl} * \frac{M(COC_2H_4COOH) - M(H)}{M(COC_2H_4COOH)} \right)
\end{aligned}
$$

$$
DS(Me) = \frac{\frac{\%\text{MeO}}{M(OCH_3)}}{\frac{\%\text{cellulose backbone}}{M(AGU)}}
\qquad
MS(HP) = \frac{\frac{\%\text{HPO}}{M(HPO)}}{\frac{\%\text{cellulose backbone}}{M(AGU)}}
$$

$$DS(Acetyl) = \frac{\frac{\%Acetyl}{M(Acetyl)}}{\frac{\%cellulose\ backbone}{M(AGU)}} \qquad DS(Succinoyl) = \frac{\frac{\%Succinoyl}{M(Succinoyl)}}{\frac{\%cellulose\ backbone}{M(AGU)}}$$

M(MeO) = M(OCH$_3$) = 31.03 Da M(HPO) = M(OCH$_2$CH(OH)CH$_3$) = 75.09 Da M(Acetyl) = M(COCH$_3$) = 43.04 Da M(Succinoyl) = M(COC$_2$H$_4$COOH) = 101.08 Da M(AGU) = 162.14 Da M(OH) = 17.008 Da M(H) = 1.008 Da

By convention, the weight percent is an average weight percentage based on the total weight of the cellulose repeat unit, including all substituents. The content of the methoxyl group is reported based on the mass of the methoxyl group (i.e., -OCH$_3$). The content of the hydroxyalkoxyl group is reported based on the mass of the hydroxyalkoxyl group (i.e., -O-alkylene-OH); such as hydroxypropoxyl (i.e., -O-CH$_2$CH(CH$_3$)-OH). The content of the aliphatic monovalent acyl groups is reported based on the mass of -C(O)-R$_1$ wherein R$_1$ is a monovalent aliphatic group, such as acetyl (-C(O)-CH$_3$). The content of the group of formula -C(O)-R-COOH is reported based on the mass of this group, such as the mass of succinoyl groups (i.e., -C(O)-CH$_2$-CH$_2$-COOH).

The esterified cellulose ethers generally have a viscosity of up to 200 mPa·s, preferably up to 100 mPa·s, more preferably up to 50 mPa·s, and most preferably up to 5.0 mPa·s, measured as a 2.0 wt.-% solution of the esterified cellulose ether in 0.43 wt.-% aqueous NaOH at 20 °C. Generally the viscosity is at least 1.2 mPa·s, more typically at least 1.8 mPa·s, even more typically at least 2.4 mPa·s, and most typically at least 2.8 mPa·s, measured as a 2.0 wt.-% solution of the esterified cellulose ether in 0.43 wt.-% aqueous NaOH at 20 °C. The 2.0 % by weight solution of the esterified cellulose ether is prepared as described in"Hypromellose Acetate Succinate, United States Pharmacopeia and National Formulary, NF 29, pp. 1548-1550", followed by an Ubbelohde viscosity measurement according to DIN 51562-1:1999-01 (January 1999).

One embodiment of the present invention is a process for fractionating an above-described esterified cellulose ether comprising groups of the formula -C(O)-R-COOH, which process comprises the steps of a) blending an esterified cellulose ether comprising groups of the formula -C(O)-R-COOH with an aqueous liquid and setting the temperature of the resulting blend to less than 10 °C to dissolve a portion of the esterified cellulose ether in the aqueous liquid, b) separating the non-dissolved portion of the esterified cellulose ether from the remainder of the blend, and c) recovering or disposing of the esterified cellulose ether that is dissolved in the aqueous liquid.

The temperature of the aqueous liquid used for preparing the blend in step a) preferably is 0 °C or more, typically 0.5°C or more. The temperature of the aqueous liquid used in step a) is typically up to 20°C, preferably less than 10 °C, more preferably less than 8°C, even more preferably less than 5 °C, and most preferably up to 3°C. Generally the esterified cellulose ether is blended with at least 5 weight parts, preferably at least 10 weight parts, more preferably at least 20 weight parts, and generally up to 100 weight parts, preferably up to 60 weight parts, more preferably up to 40 weight parts, of aqueous liquid per weight part of esterified cellulose ether.

It is essential in the process of the present invention that the temperature of the resulting blend in step a) is set to less than 10°C, preferably less than 8°C, more preferably less than 5 °C, and most preferably to 3 °C or less. The temperature of the resulting blend is generally set to at least minus 2°C, typically to 0 °C or more, and more typically to 0.5°C or more. It is not essential whether the temperature of the aqueous liquid is adjusted before or after blending with the esterified cellulose ether. Preferably the blend is left at the above-mentioned temperature for a time period of up to a week, more preferably up to 72 hours, and more preferably up to 24 hours. Preferably the blend is left at the above-mentioned temperature for a time period of at least 10 minutes, preferably at least 30 minutes, and more preferably at least 2 hours.

The aqueous liquid may additionally comprise a minor amount of an organic liquid diluent; however, the aqueous liquid should generally comprise at least 80, preferably at least 85, more preferably at least at least 90, and particularly at least 95 weight percent of water, based on the total weight of the aqueous liquid. The term "organic liquid diluent" as used herein means an organic solvent or a mixture of two or more organic solvents. Preferred organic liquid diluents are polar organic solvents having one or more heteroatoms, such as oxygen, nitrogen or halogen like chlorine. More preferred organic liquid diluents are alcohols, for example multifunctional alcohols, such as glycerol, or preferably monofunctional alcohols, such as methanol, ethanol, isopropanol or n-propanol; ethers, such as tetrahydrofuran, ketones, such as acetone, methyl ethyl ketone, or methyl isobutyl ketone; acetates, such as ethyl acetate; halogenated hydrocarbons, such as methylene chloride; or nitriles, such as acetonitrile. More preferably the organic liquid diluents have 1 to 6, most preferably 1 to 4 carbon atoms. The aqueous liquid may comprise a basic compound, but the degree of neutralization of the groups -C(O)-R-COOH of the esterified cellulose ether in the resulting blend of esterified cellulose ether and aqueous liquid should not be more than 0.4, preferably not more than 0.3 or 0.2 or 0.1, more preferably not more than 0.05 or 0.01, and most preferably not more than 10$^{-3}$ or even not more than 10$^{-4}$. The term "degree of neutralization" as used herein defines the ratio of deprotonated carboxylic groups over the sum of deprotonated and protonated carboxylic groups, i.e.,

Degree of neutralization = [-C(O)-R-COO$^-$] / [-C(O)-R-COO$^-$ + -C(O)-R-COOH]. Preferably the aqueous liquid does not comprise a substantial amount of a basic compound. More preferably, the aqueous liquid does not contain a basic compound. Most preferably, the aqueous liquid comprises from 80 to 100 percent, preferably 85 to 100 percent, more preferably 90 to 100 percent and most preferably 95 to 100 percent of water, and from 0 to 20 percent, preferably 0 to 15 percent, more preferably 0 to 10 percent, and most preferably 0 to 5 percent of an organic liquid diluent, based on the total weight of the aqueous liquid. Most preferably the aqueous liquid consists of water, e.g., deionized or distilled water.

Surprisingly, a portion of the esterified cellulose ether comprising groups of the formula -C(O)-R-COOH is soluble in the aqueous liquid under the above-mentioned temperature conditions, even when the esterified cellulose ether in the aqueous liquid has a degree of neutralization of the groups -C(O)-R-COOH of not more than 0.4 or a preferred range listed above, e.g., when the esterified cellulose ether is blended with only water, such as deionized or distilled water. Those esterified cellulose ethers comprising groups of the formula -C(O)-R-COOH that are soluble in the above-mentioned blend and those that are insoluble in the above-mentioned blend at a temperature of less than 10 °C have very different molecular weights as will be described in more details below.

In step b) of the fractionation process of the present invention the non-dissolved portion of the esterified cellulose ether can be separated from the remainder of the blend in a known manner, such as by centrifugation or filtration or upon settling by decantation. The portion of the esterified cellulose ether that remains non-dissolved in the blend at a temperature of less than 10 °C generally has a weight average molecular weight $M_w$ of at least 80,000 Dalton, preferably at least 100,000 Dalton, and more preferably at least 150,000 Dalton. This esterified cellulose ether generally has a weight average molecular weight $M_w$ of up to 500,000 Dalton, preferably up to 350,000 Dalton, and more preferably up to 300,000 Dalton. The portion of the esterified cellulose ether that remains non-dissolved in the blend at a temperature of less than 10 °C preferably has a ratio of weight average molecular weight $M_w$ to number average molecular weight $M_n$, i.e., a polydispersity $M_w/M_n$, of not more than 2.6, more preferably not more than 2.4, most preferably not more than 2.3, and particularly not more than 2.0. The polydispersity $M_w/M_n$ generally is at least 1.3, typically at least 1.6, and more typically at least 1.8. It has been found that the portion of the esterified cellulose ether that remains non-dissolved in the blend at a temperature of less than 10 °C and that has the above-mentioned properties enhances the bioavailability of poorly water-soluble drugs to a larger extent than the esterified cellulose ether which is the starting material in the process of the present invention.

After separation of the non-dissolved esterified cellulose ether from the remainder of the blend at a temperature of less than 10 °C as described above, the aqueous liquid surprisingly still comprises dissolved esterified cellulose ether. The portion of the dissolved esterified cellulose ether is generally at least 1 percent, typically at least 5 percent, and generally up to 70 percent, typically up to 50 percent, based on the total weight of the esterified cellulose ether. The dissolved esterified cellulose ether is invisible to the naked eye. If the commercial interest only lies in obtaining a water-insoluble esterified cellulose ether of high molecular weight and high ability to enhances the bioavailability of poorly water-soluble drugs, the esterified cellulose ether that is dissolved in the aqueous liquid can be disposed of, e.g. by recycling in step c) of the process of the present invention. However, in a preferred embodiment of the invention the esterified cellulose ether that is dissolved in the aqueous liquid is recovered in step c) of the fractionation process of the present invention, e.g., by heating the aqueous liquid comprising the dissolved esterified cellulose ether to a temperature of at least 30 °C, preferably at least 45 °C more preferably at least 60 °C, and most preferably at least 80 °C. Typically the aqueous liquid comprising the dissolved esterified cellulose ether is heated to a temperature of up to 98 °C, more typically of up to 95 °C. At such temperatures the dissolved esterified cellulose ether precipitates. The precipitated esterified cellulose ether can be separated from the aqueous liquid in a known manner, such as by centrifugation or filtration or upon settling by decantation. The observed water-insolubility of this esterified cellulose ether upon heating is reversible. The separated esterified cellulose ether is soluble in an aqueous liquid at a temperature of less than 10 °C. Alternatively, the esterified cellulose ether that is dissolved in the aqueous liquid is recovered in step c) of the fractionation process of the present invention by another known technique, such as freeze-drying or spray-drying.

The esterified cellulose ether obtained in step c) of the process of the present invention is soluble in an aqueous liquid at a temperature of less than 10 °C. Generally least 85 wt.%, typically at least 90 wt.%, more typically at least 95 wt.%, and in most cases at least 99 wt.% of the esterified cellulose ether is soluble in a mixture of 2.5 weight parts of the esterified cellulose ether and 97.5 weight parts of water at 5 °C. Typically this degree of solubility is also observed in a mixture of 5 or 10 weight parts of the esterified cellulose ether and 95 or 90 weight parts of water at 5 °C or even in a mixture of 20 weight parts of the esterified cellulose ether and 80 weight parts of water at 5 °C.

The water-soluble esterified cellulose ether obtained in step c) generally has a weight average molecular weight $M_w$ of at least 8,000 Dalton, preferably at least 10,000 Dalton, and more preferably at least 11,000 Dalton or at least 12,000 Dalton. The water-soluble esterified cellulose ether generally has a weight average molecular weight $M_w$ of up to 70,000 Dalton, preferably up to 60,000 Dalton, and more preferably up to 50,000 Dalton or up to 40,000 Dalton. The water-soluble esterified cellulose ether generally has a polydispersity $M_w/M_n$, i.e., a ratio of weight average molecular weight $M_w$ to number average molecular weight $M_n$, of not more than 2.6, preferably not more than 2.3, more preferably not more than 2.1, and in some embodiments even not more than 1.5. The polydispersity $M_w/M_n$ generally is at least 1.1, typically at least 1.2, and more typically at least 1.3.

$M_w$ and $M_n$ are measured according to Journal of Pharmaceutical and Biomedical Analysis 56 (2011) 743 using a mixture of 40 parts by volume of acetonitrile and 60 parts by volume of aqueous buffer containing 50 mM $NaH_2PO_4$ and 0.1 M $NaNO_3$ as mobile phase. The mobile phase is adjusted to a pH of 8.0. The measurement of $M_w$ and $M_n$ is described in more details in the Examples.

The process of the present invention for fractionating an esterified cellulose ether allows the production of at least one fraction of an esterified cellulose ether which has a low polydispersity. This is highly desirable because a low polydispersity, i.e a low ratio of weight average molecular weight $M_w$ to number average molecular weight $M_n$, $M_w/M_n$, of the esterified cellulose ether is an indication of a fairly tight molecular weight distribution. High tightness of molecular weight distribution is desirable for polymers that act as excipients in pharmaceutical dosage forms in order to increase reproducibility of the properties of individual dosage forms and to increase the uniformity of the interaction of the polymer molecules with the active ingredient, which maximizes the predictability of the efficiency of the dosage forms.

Another embodiment of the present invention is a process for preparing an esterified cellulose ether comprising groups of the formula -C(O)-R-COOH, wherein R is a divalent hydrocarbon group, which process comprises the steps of a) reacting a cellulose ether with a dicarboxylic acid anhydride or with a combination of a dicarboxylic acid anhydride and an aliphatic monocarboxylic acid anhydride in the presence of an aliphatic carboxylic acid to produce a reaction product mixture comprising an esterified cellulose ether comprising groups of the formula -C(O)-R-COOH, precipitating the esterified cellulose ether from the reaction product mixture, blending the precipitated esterified cellulose ether with an aqueous liquid and setting the temperature of the resulting blend to less than 10 °C to dissolve a portion of the esterified cellulose ether in the aqueous liquid, b) separating the non-dissolved portion of the esterified cellulose ether from the remainder of the blend, and c) recovering or disposing of the esterified cellulose ether that is dissolved in the aqueous liquid.

The reaction of a cellulose ether with a dicarboxylic acid anhydride or with a combination of a dicarboxylic acid anhydride and an aliphatic monocarboxylic acid anhydride in the presence of an aliphatic carboxylic acid to produce a reaction product mixture comprising an esterified cellulose ether can be conducted in a known manner, for example as described in U.S. Patent Nos. 3,435,027 and 4,226,981, in the International Patent Applications WO 2005/115330 or WO2013/148154, or in European Patent Application EP 0 219 426.

A preferred dicarboxylic acid anhydride is succinic anhydride. Preferred aliphatic monocarboxylic acid anhydrides are selected from the group consisting of acetic anhydride, butyric anhydride and propionic anhydride. The molar ratio between the anhydride of a dicarboxylic acid and the anhydroglucose units of cellulose ether generally is 0.1 or more, and preferably 0.2 or more. The molar ratio between the anhydride of a dicarboxylic acid and the anhydroglucose units of cellulose ether generally is 1.5 or less, and preferably 1 or less. If an anhydride of an aliphatic monocarboxylic acid is used, the molar ratio between the anhydride of an aliphatic monocarboxylic acid and the anhydroglucose units of the cellulose ether generally is at least 0.1/1, preferably at least 0.3/1, more preferably at least 0.5/ 1, most preferably at least 1/1 and particularly at least 1.5/1; and up 17/1, preferably up to 10 /1, more preferably up to 8/ 1, most preferably up to 6/1, and particularly up to 4/1.

The esterification step is conducted in an aliphatic carboxylic acid, such as acetic acid, propionic acid, or butyric acid. The reaction diluent can comprise minor amounts of other solvents or diluents which are liquid at room temperature and do not react with the cellulose ether, such as aromatic or aliphatic solvents like benzene, toluene, 1,4-dioxane, or tetrahydrofurane; or halogenated $C_1$-$C_3$ derivatives, like dichloro methane or dichloro methyl ether, but the amount of the aliphatic carboxylic acid is more than 50 percent, more preferably at least 75 percent, and even more preferably at least 90 percent, based on the total weight of the reaction diluent. Most preferably the reaction diluent consists of an aliphatic carboxylic acid. The esterification reaction is generally conducted in the presence of 100 to 2,000 parts by weight of an aliphatic carboxylic acid as the reaction medium per 100 parts by weight of the cellulose ether. The molar ratio [aliphatic carboxylic acid / anhydroglucose units of cellulose ether] generally is from [3.8/1] to [15/1], preferably from [4/1] to [12/1], and more preferably from [4.9/1.0] to [11.5 /1.0].

The esterification reaction is generally conducted in the presence of an esterification catalyst, preferably in the presence of an alkali metal carboxylate, such as sodium acetate or potassium acetate. The amount of the alkali metal carboxylate is preferably 20 to 200 parts by weight of the alkali metal carboxylate per 100 parts by weight of the cellulose ether. The molar ratio [alkali metal carboxylate / anhydroglucose units of cellulose ether] is preferably from [0.4 / 1.0] to [3.8/ 1.0], more preferably from [1.5 / 1.0] to [3.5/ 1.0], and most preferably from [1.9/ 1.0] to [3.0/ 1.0].

The reaction temperature for the esterification is generally 60°C or more, and preferably 70°C or more. The reaction temperature is generally up to 110°C, preferably up to 100°C. The esterification reaction is typically completed within 2 to 25 hours, more typically within 2 to 8 hours.

The resulting reaction product mixture comprises the esterified cellulose ether, typically an aliphatic carboxylic acid used as a reaction medium, typically a reaction catalyst, such as an alkali metal carboxylate, typically residual amounts of one or more esterification agents and by-products, such as a dicarboxylic acid anhydride and optionally an aliphatic monocarboxylic acid anhydride. The reaction product mixture generally comprises from 3 to 60 weight percent, typically from 7 to 35 weight percent of the esterified cellulose ether, based on the total weight of the reaction product mixture. The amount of the aliphatic carboxylic acid in the reaction product mixture generally is from 10 to 95 weight percent, typically from 20 to 70 weight percent, based on the total weight of the reaction product mixture. The amount of the reaction catalyst, such as an alkali metal carboxylate, generally is from 1 to 50 weight percent, typically from 5 to 30 weight percent, based on the total weight of the reaction product mixture. The reaction product mixture generally comprises from 0.1 to 50, typically from 2 to 40 weight percent of minor components, such as a non-reacted dicarboxylic acid anhydride and optionally a non-reacted aliphatic monocarboxylic acid anhydride. The reaction product mixture comprising the esterified cellulose ether generally has a temperature of 60 °C or more, typically of 75 °C or more, and generally up to 110 °C, typically up to 90 °C.

After completion of the esterification reaction, the esterified cellulose ether is precipitated from the resulting reaction product mixture. The esterified cellulose ether can be precipitated from the reaction mixture in a known manner, for example as described in U.S. Patent No. 4,226,981, International Patent Application WO 2005/115330, European Patent Application EP 0 219 426 or International Patent Application WO2013/148154.

The precipitated esterified cellulose ether is subsequently washed with an aqueous liquid. Suitable aqueous liquids are described further above. In the washing step the precipitated esterified cellulose ether is blended with an aqueous liquid; preferably 2 to 400 weight parts, more preferably 3 to 300 weight parts, and most preferably 4 to 150 weight parts of aqueous liquid are used per weight part of esterified cellulose ether. The washing step can be repeated once or several times, preferably once to 5 times. Surprisingly, it has been found that a portion of the precipitated esterified cellulose ether is dissolved in the aqueous liquid used for washing purposes when the blend of esterified cellulose ether and aqueous liquid is set to a temperature of less than 10 °C, preferably to less than 8 °C, more preferably to less than 5 °C, and most preferably to 3 °C or less. This finding allows to conduct the above described fractionation process during the washing of the esterified cellulose ether after its precipitation from the reaction mixture, i.e., the fractionation process can be integrated into the process for producing the esterified cellulose ethers.

In step b) of the production process of the present invention the non-dissolved portion of the esterified cellulose ether, i.e., the portion of the esterified cellulose ether that does not dissolve in the blend of esterified cellulose ether and aqueous liquid at a temperature of less than 10 °C, can be separated from the remainder of the blend as described above in step b) of the fractionation process. The separated non-dissolved portion of the esterified cellulose has the properties as described in step b) of the fractionation process described above, e.g., generally a weight average molecular weight $M_w$ of at least 80,000 Dalton.

In step c) of the production process of the present invention the esterified cellulose ether that is dissolved in the aqueous liquid is recovered or disposed of as described above in step b) of the fractionation process. The esterified cellulose ether obtained in step c) of the process of the present invention is soluble in an aqueous liquid at a temperature of less than 10 °C. It has the properties as described in step c) of the fractionation process described above, e.g., a weight average molecular weight $M_w$ of up to 70,000 Dalton.

Some embodiments of the invention will be described in detail in the Examples. Unless otherwise mentioned, all parts and percentages are by weight. In the Examples the following test procedures are used.

EXAMPLES

Content of ether and ester groups

The content of ether groups in the esterified cellulose ether is determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) are determined according to Hypromellose Acetate Succinate, United States Pharmacopia and National Formulary, NF 29, pp. 1548-1550". Reported values for ester substitution are corrected for volatiles (determined as described in section "loss on drying" in the above HPMCAS monograph).

Viscosity of Hydroxypropyl Methyl Cellulose Acetate Succinate (HPMCAS)

The 10 wt.-% solution of HPMCAS in acetone was prepared by mixing 10.0 g HPMCAS, based on its dry weight, with 90.0 g of acetone under vigorous stirring at room temperature. The mixture was rolled on a roller mixer for about 24 hours. The solution was centrifuged at 2000 rpm for 3 minutes using a Megafuge 1.0 centrifuge, commercially available from Heraeus Holding GmbH, Germany. An Ubbelohde viscosity measurement according to DIN 51562-1:1999-01 (January 1999) was carried out. The measurement was done at 20 °C.

Determination of $M_w$, $M_n$ and $M_z$

Mw, Mn and Mz are measured according to Journal of Pharmaceutical and Biomedical Analysis 56 (2011) 743-747 unless stated otherwise. The mobile phase was prepared by mixing mixture of 40 parts by volume of acetonitrile and 60 parts by volume of aqueous buffer containing 50 mM $NaH_2PO_4$ and 0.1 M NaNO3. The mobile phase was adjusted to a pH of 8.0. Solutions of the cellulose ether esters were filtered into a HPLC vial through a syringe filter of 0.45 $\mu$m pore size.

More specifically, the utilized Chemicals and solvents were:
Polyethylene oxide standard materials (abbreviated as PEOX 20 K and PEOX 30 K) were purchased from Agilent Technologies, Inc. Palo Alto, CA, catalog number PL2083-1005 and PL2083-2005.

Acetonitrile (HPLC grade $\geq$ 99.9 %, CHROMASOL plus), catalog number 34998, sodium hydroxide (semiconductor grade, 99.99 %, trace metal base), catalog number 306576, water (HPLC grade, CHROMASOLV Plus) catalog number 34877 and sodium nitrate (99,995 %, trace metal base) catalog number 229938 were purchased from Sigma-Aldrich, Switzerland.

Sodium dihydrogen phosphate ($\geq$ 99.999 % TraceSelect) catalog number 71492.was purchased from FLUKA, Switzerland.

The normalization solution of PEOX20 K at 5 mg/mL, the standard solution of PEOX30 K at 2 mg/mL, and the sample solution of HPMCAS at 2 mg/mL were prepared by adding a weighed amount of polymer into a vial and dissolving it with a measured volume of mobile phase. All solutions were allowed to dissolve at room temperature in the capped vial for 24 h with stirring using a PTFE-coated magnetic stirring bar.

The normalization solution (PEOX 20k, single preparation, N) and the standard solution (PEOX30 K, double preparation, S1 and S2) were filtered into a HPLC vial through a syringe filter of 0.02 $\mu$m pore size and 25 mm diameter (Whatman Anatop 25, catalog number 6809-2002), Whatman.

The test sample solution (HPMCAS, prepared in duplicate, T1, T2) and a laboratory standard (HPMCAS, single preparation, LS) were filtered into a HPLC vial through a syringe filter of 0.45 $\mu$m pore size (Nylon, e.g. Acrodisc 13 mm VWR catalog number 514-4010).

Chromatographic condition and run sequence were conducted as described by Chen, R. et al.; Journal of Pharmaceutical and Biomedical Analysis 56 (2011) 743- 748). The SEC-MALLS instrument set-up included a HP1100 HPLC system from Agilent Technologies, Inc. Palo Alto, CA; a DAWN Heleos II 18 angle laser light scattering detector and a OPTILAB rex refractive index detector, both from Wyatt Technologies, Inc. Santa Barbara, CA. The analytical size exclusion column (TSK-GEL® GMPWXL, 300 × 7.8 mm) was purchased from Tosoh Bioscience. Both the OPTILAB and the DAWN were operated at 35°C. The analytical SEC column was operated at room temperature (24 $\pm$ 5 °C). The mobile phase was a mixture of 40 volume parts of acetonitrile and 60 volume parts of aqueous buffer containing 50 mM $NaH_2PO_4$ and 0.1 M NaNO3 prepared as follows:
Aqueous buffer: 7.20 g of sodium dihydrogen phosphate and 10.2 g of sodium nitrate were added to 1.2 L purified water in a clean 2 L glass bottle under stirring until dissolution.

Mobile phase: 800 mL of acetonitrile were added to 1.2 L of the aqueous buffer prepared above, and stirred until a good mixture was achieved and the temperature equilibrated to ambient temperature.

The mobile phase was pH adjusted to 8.0 with 10M NaOH and filtered through a 0.2 m nylon membrane filter. The flow rate was 0.5 mL/min with in-line degassing. The injection volume was 100 $\mu$L and the analysis time was 35 min.

The MALLS data were collected and processed by Wyatt ASTRA software (version 5.3.4.20) using dn/dc value (refractive index increment) of 0.120 mL/g for HPMCAS. The light scattering signals of detector Nos. 1-4, 17, and 18) were not used in the molecular weight calculation. A representative chromatographic run sequence is given below: B, N, LS, S1 (5x), S2, T1 (2x), T2 (2x), T3 (2x), T4 (2x), S2, T5(2x), etc., S2, LS, W, where, B represents blank injection of mobile phase, N1 represents normalization solution; LS represents a laboratory standard HPMCAS; S1 and S2 represent standard solutions one and two, respectively; T1, T2, T3, T4, and T5 represent test sample solutions and W represents water injection. (2x) and (5x) denote the number of injections of the same solution.

Both the OPTILAB and the DAWN were calibrated periodically according to the manufacturer's recommended procedures and frequency. A 100 $\mu$L injection of a 5 mg/mL polyethylene oxide standard (PEOX20 K) was employed for normalizing all angle light scattering detectors relative to 90° detector for each run sequence.

Use of this mono-dispersed polymer standard also enabled the volume delay between the OPTILAB and the DAWN to be determined, permitting proper alignment of the light scattering signals to the refractive index signal. This is necessary for the calculation of the weight-averaged molecular weight (Mw) for each data slice.

Example 1

A hydroxypropyl methyl cellulose acetate succinate (HPMCAS-I) was produced in a known manner by reacting a hydroxypropyl methylcellulose (HPMC) with acetic anhydride and succinic anhydride in the presence of glacial acetic acid and sodium acetate (water free). The HPMC contained 28.4 % methoxyl groups, 9.0 % hydroxypropoxyl groups and a viscosity of 3 mPa·s, measured as a 2 % solution in water at 20 °C according to ASTM D2363 - 79 (Reapproved 2006). The HPMC is commercially available from The Dow Chemical Company as Methocel E3 LV Premium cellulose ether. The produced HPMCAS-I was purified several times with water having a temperature of 23°C. Totally 100 weight parts of water were used per 1 weight part of HPMCAS-I.

344 g of HPMCAS-I having a temperature of 20°C was suspended in 2.83 liter of water having a temperature of 2°C under stirring for 2h and stored for 12h at 0.5 °C. The resulting blend of HPMCAS-I and water had a temperature of 0.5 °C. Then the liquid portion of the blend was separated from the suspended HPMCAS by centrifugation (Microfuge 1.0, Heraeus, 4000 rpm, 5min) at a temperature of 5 °C.

The HPMCAS that remained un-dissolved in the blend of HPMCAS-I and water is designated hereafter as HPMCAS-IA

A portion of the HPMCAS-I was dissolved in the blend of HPMCAS-I and water. This water-soluble portion is designated hereafter as HPMCAS-IB. The water soluble HPMCAS-IB was precipitated from the liquid by heating the liquid to 95°C for 10 min. It was 14 % of the total amount of HPMCAS-I.

The properties of the starting material HPMCAS-I and of the HPMCAS-IA and HPMCAS-IB, that were recovered after the suspension of HPMCAS-I in water at a temperature of less than 8 °C as described above, are listed in Table 1 below.

Example 2

In another experiment a hydroxypropyl methyl cellulose acetate succinate (HPMCAS-II) was produced in a known manner by reacting a hydroxypropyl methylcellulose (HPMC) with acetic anhydride and succinic anhydride in the presence of glacial acetic acid and sodium acetate (water free). The HPMC contained 28.7 % methoxyl groups, 9.0 % hydroxypropoxyl groups and a viscosity of 3 mPa·s, measured as a 2 % solution in water at 20 °C according to ASTM D2363 - 79 (Reapproved 2006). The HPMC is commercially available from The Dow Chemical Company as Methocel E3 LV Premium cellulose ether. The produced HPMCAS-II was purified several times with water having a temperature of 23°C. Totally 100 weight parts of water were used per 1 weight part of HPMCAS-II.

100 g of HPMCAS-II having a temperature of 20°C was suspended in 5 liter of water having a temperature of 1.5°C under stirring for 4 h. The resulting blend of HPMCAS-II and water had a temperature of 2°C. Then the liquid portion of the blend was separated from the suspended HPMCAS by filtration over a metal sieve at a temperature of 5 °C. Afterwards the remaining solid was washed several times using totally 48 liter of water having a temperature of 1.5°C. The resulting blend had a temperature of 2°C. Then the liquid portion of the blend was separated from the suspended HPMCAS by filtration at a temperature of 5 °C.

The HPMCAS that remained un-dissolved in the blend of HPMCAS-II and water is designated hereafter as HPMCAS-IIA.

A portion of the HPMCAS-II was dissolved in the blend of HPMCAS-II and water. This water-soluble portion is designated hereafter as HPMCAS-IIB. The water soluble HPMCAS-IIB was recovered as solid mass from the first filtration liquid by freeze-drying. It was 9 % of the total amount of HPMCAS-II.

The properties of the starting material HPMCAS-II and of the HPMCAS-IIA and HPMCAS-IIB, that were recovered after suspension of HPMCAS-II in water at a temperature of less than 8 °C as described above, are listed in Table 1 below.

Example 3

In another experiment a hydroxypropyl methyl cellulose acetate succinate (HPMCAS-III) was produced in a known manner by reacting a hydroxypropyl methylcellulose (HPMC) with acetic anhydride and succinic anhydride in the presence of glacial acetic acid and sodium acetate (water free). The same HPMC was used as in Example 2.

750g of HPMCAS-III having a temperature of 20 °C was suspended in 4.6 liter of water having a temperature of 2°C under stirring for 2h and stored for 12h at 3°C. The resulting blend of HPMCAS-III and water had a temperature of 3 °C. Then the liquid portion of the blend was separated from the suspended HPMCAS by centrifugation (Microfuge 1.0, Heraeus, 10000 rpm, 20min) at a temperature of 1 °C.

The HPMCAS that remained un-dissolved in the blend of HPMCAS-III and water is designated hereafter as HPMCAS-IIIA.

A portion of the HPMCAS-III was dissolved in the blend of HPMCAS-III and water. This water-soluble portion is designated hereafter as HPMCAS-IIIB. The water soluble HPMCAS-IIIB was recovered as solid mass from the liquid by freeze-drying. 75 g of water soluble HPMCAS-IIIB was recovered (10 % of the total weight of HPMCAS-III).

The properties of the starting material HPMCAS-III and of the HPMCAS-IIIA and HPMCAS-IIIB, that were recovered after the suspension of HPMCAS-III in water at a temperature of less than 8 °C as described above, are listed in Table 1 below.

The results in Table I below illustrate that esterified cellulose ethers are obtained by the process of the present invention which have a high weight average molecular weight $M_w$ and which have a lower $M_w/M_n$ than the esterified cellulose ethers that are used as starting material in the process of the present invention, as illustrated by HPMCAS-IA, HPMCAS-IIA and HPMCAS-IIIA. When the esterified cellulose ethers used as a starting material already have a low $M_w/M_n$, the additional reduction in $M_w/M_n$ is understandably smaller.

Moreover, the results in Table II below illustrate that the obtained esterified cellulose ethers which have a high weight average molecular weight $M_w$ and which have a lower $M_w/M_n$ than the esterified cellulose ethers that are used as starting material in the process of the invention have a higher ability to enhance the bioavailability of poorly water-soluble drugs, as compared to the esterified cellulose ethers that are used as starting material in the process. Enhanced bioavailability of poorly water-soluble drugs may be determined through either *in vitro* dissolution tests or through *in vivo* tests. It has been determined that enhanced drug concentrations in *in vitro* dissolution tests provide good indicators of *in vivo* performance and bioavailability.

Table 1

| HPMCAS | Molecular weight (kDA) | | | | 10% viscosity in acetone [mPa·s] | Ether Substitution | | Ester substitution | | Ether Substitution | | Ester substitution | |
|---|---|---|---|---|---|---|---|---|---|---|---|---|---|
| | $M_w$ | $M_n$ | $M_w/M_n$ | Recovery Rate (%) | | Methoxyl (%) | Hydroxypropoxyl (%) | Acetyl (%) | Succinoyl (%) | $DS_M$ | $MS_{HP}$ | $DOS_{AC}$ | DOSs |
| HPMCAS-I | 186 | 92 | 2.0 | 94.8 | 19.5 | 23.4 | 7.5 | 11.8 | 7.3 | 1.88 | 0.25 | 0.69 | 0.18 |
| HPMCAS-IA | 240 | 126 | 1.9 | 95.0 | 25 | 23.5 | 7.6 | 11.7 | 7.5 | 1.90 | 0.25 | 0.68 | 0.19 |
| HPMCAS-IB** | 63 | 29 | 2.2 | 96.6 | 6.4 | 26.0 | 7.4 | 11.2 | 6.2 | 2.08 | 0.24 | 0.64 | 0.15 |
| HPMCAS-II | 152 | 68 | 2.2 | 97.8 | 23 | 23.2 | 7.3 | 9.7 | 11.2 | 1.91 | 0.25 | 0.57 | 0.28 |
| HPMCAS-IIA | 202 | 106 | 1.9 | 93.9 | 41 | 22.7 | 7.5 | 10.0 | 11.7 | 1.89 | 0.26 | 0.60 | 0.30 |
| HPMCAS-IIB | 13 | 10 | 1.3 | 96.3 | n.a. | 26.1 | 7.0 | 8.7 | 8.6 | 2.07 | 0.23 | 0.50 | 0.21 |
| HPMCAS-III | 114 | 46 | 2.5 | 100 | 18 | 22.8 | 7.2 | 8.0 | 14.5 | 1.92 | 0.25 | 0.49 | 0.38 |
| HPMCAS-IIIA | 126 | 54 | 2.3 | 99.8 | n.a. | 22.4 | 7.3 | 8.0 | 14.9 | 1.90 | 0.26 | 0.49 | 0.39 |
| HPMCAS-IIIB | 13.6 | 11.1 | 1.2 | 100 | 3.3 | 25.6 | 6.8 | 7.1 | 11.2 | 2.05 | 0.23 | 0.41 | 0.28 |
| ** average of 2 measurements   n.a.: not assessed | | | | | | | | | | | | | |

Impact of HPMCAS on the Aqueous Solubility of a Poorly Soluble Drug

The ability of HPMCAS-II and HPMCAS-IIA to maintain drug concentrations in an aqueous solution at supersaturation levels was tested with the poorly water soluble drug Torcetrapib.

Torcetrapib has a water solubility of 0.00013 $\mu$g/ml.

Spray-dried dispersions (SDDs) comprising 25 wt% Torcetrapib in HPMCAS-II and HPMCAS-IIA respectively were prepared. A spray solution was prepared by dissolving 50 mg of Torcetrapib and 150 mg of the respective HPMCAS in 9.8 g of acetone. This solution was spray-dried using a custom-made bench top spray-dryer, which consisted of an atomizer in the top cap of a vertically oriented 11-cm diameter steel pipe. The atomizer was a two-fluid nozzle (Spraying Systems Co. Model 1650 fluid cap and 64 air cap), where the atomizing gas was nitrogen delivered to the nozzle at 65 °C and a flow rate of 1.1 SCFM (Standard Cubic Feet per minute), corresponding to 31.1 liters per minute, and the solution to be spray-dried was delivered to the nozzle at room temperature and at a flow rate of 1.3 mL/min using a syringe pump. Filter paper with a supporting screen was clamped to the bottom end of the pipe to collect the solid spray-dried material and allow the nitrogen and evaporated solvent to escape.

The SDDs were evaluated in duplicate by microcentrifuge dissolution at 37°C in phosphate buffered saline (PBS) comprising 20.00 mM sodium phosphate ($Na_2HPO_4$), 46.69 mM potassium phosphate ($KH_2PO_4$), 84.57 mM NaCl, and 0.07 mM KCl, adjusted to pH 6.5 with dilute aqueous NaOH, to which was added 0.5wt% SIF powder (Biorelevant, Surrey, UK) to prepare a simulated fasted solution. The following *in vitro* dissolution test was performed on the SDDs to determine the concentration enhancement caused by HPMCAS-II and HPMCAS-IIA respectively, as generally described in MOLECULAR PHARMACEUTICS, VOL. 5, NO.6, 1003-1019.

A sufficient amount of material was added to a microcentrifuge test tube to achieve the desired dose (1000 $\mu$g/ml) if all the drug dissolved. The tubes were placed in a 37 °C temperature-controlled chamber, and 1.8 mL of a solution of PBS was added to each tube. The samples were quickly mixed using a vortex mixer for about 60 s. At each time point, the samples were centrifuged at 13 000g at 37 °C for 1 min. The supernatant solution was sampled and diluted 1:6 (by volume) with methanol and then analyzed by HPLC. Following sampling, the contents of each tube were mixed on the vortex mixer for about 60 s and then allowed to stand.

The dissolution properties of Torcetrapib in HPMCAS-IIA were measured and compared to the dissolution properties of Torcetrapib in HPMCAS-II. The results are illustrated in Table 2.

Table 2

| x min. | SDD of Torcetrapib in HPMCAS-**II**, drug concentration at x min. [mg/L] | SDD of Torcetrapib in HPMCAS-**IIA**, drug concentration at x min. [mg/L] |
|---|---|---|
| 4 | 689 | 592 |
| 10 | 772 | 836 |
| 20 | 736 | 796 |
| 40 | 671 | 746 |
| 90 | 567 | 655 |
| 180 | 458 | 558 |
| 360 | 363 | 447 |
| | | |
| $AUC_{90}$ | $AUC_{90}$ = 58337 min*$\mu$g/ml | $AUC_{90}$ = 64080 min*$\mu$g/ml |
| $AUC_{90}$: area under the curve from 0-90 minutes | | |

**Claims**

1. A process for fractionating an esterified cellulose ether comprising groups of the formula -C(O)-R-COOH, wherein R is a divalent hydrocarbon group, which process comprises the steps of

   a) blending the esterified cellulose ether comprising groups of the formula -C(O)-R-COOH with an aqueous liquid and setting the temperature of the resulting blend to less than 10 °C to dissolve a portion of the esterified cellulose ether in the aqueous liquid,
   b) separating the non-dissolved portion of the esterified cellulose ether from the remainder of the blend, and

c) recovering or disposing of the esterified cellulose ether that is dissolved in the aqueous liquid.

2. A process for preparing an esterified cellulose ether comprising groups of the formula -C(O)-R-COOH, wherein R is a divalent hydrocarbon group, which process comprises the steps of

   a) reacting a cellulose ether with a dicarboxylic acid anhydride or with a combination of a dicarboxylic acid anhydride and an aliphatic monocarboxylic acid anhydride in the presence of an aliphatic carboxylic acid to produce a reaction product mixture comprising an esterified cellulose ether comprising groups of the formula -C(O)-R-COOH, precipitating the esterified cellulose ether from the reaction product mixture, blending the precipitated esterified cellulose ether with an aqueous liquid and setting the temperature of the resulting blend to less than 10 °C to dissolve a portion of the esterified cellulose ether in the aqueous liquid,
   b) separating the non-dissolved portion of the esterified cellulose ether from the remainder of the blend, and
   c) recovering or disposing of the esterified cellulose ether that is dissolved in the aqueous liquid.

3. The process of claim 1 or 2 wherein step a) comprises setting the temperature of the resulting blend to a temperature of less than 8 °C.

4. The process of claim 1 or 2 wherein step a) comprises setting the temperature of the resulting blend to a temperature of less than 5 °C.

5. The process of any one of claims 1 to 4 wherein step a) comprises blending the esterified cellulose ether with the aqueous liquid at a weight ratio of from 5 to 100 weight parts of aqueous liquid per one weight part of esterified cellulose ether.

6. The process of any one of claims 1 to 5 wherein in step c) the esterified cellulose ether is recovered by heating the aqueous liquid comprising dissolved esterified cellulose ether to a temperature of at least 30 °C or by freeze-drying.

7. The process of any one of claims 1 to 6 wherein the esterified cellulose ether comprises groups of the formula -C(O)-CH2-CH2-COOH.

8. The process of any one of claims 1 to 7 wherein the esterified cellulose ether additionally comprises aliphatic monovalent acyl groups.

9. The process of any one of claims 1 to 8 wherein the esterified cellulose ether is hydroxypropyl methylcellulose acetate succinate.

10. The process of any one of claims 1 to 9 wherein an esterified cellulose ether having a weight average molecular weight Mw of at least 80,000 Dalton and a Polydispersity Mw/Mn of not more than 2.6 is obtained in step b).

11. The process of any one of claims 1 to 10 wherein an esterified cellulose ether having a weight average molecular weight Mw of up to 70,000 Dalton and a Polydispersity Mw/Mn of not more than 2.6 is obtained in step c).

**Patentansprüche**

1. Ein Verfahren zur Fraktionierung eines veresterten Celluloseethers, welcher Gruppen der Formel -C(O)-R-COOH enthält, worin R eine divalente Kohlenwasserstoffgruppe ist, wobei das Verfahren die Schritte umfasst

   a) Mischen des veresterten Celluloseethers, welcher Gruppen der Formel -C(O)-R-COOH umfasst, mit einer wässrigen Flüssigkeit und Einstellen der Temperatur der resultierenden Mischung auf weniger als 10°C, um einen Teil des veresterten Celluloseethers in der wässrigen Flüssigkeit zu lösen,
   b) Trennen des nichtgelösten Teils des veresterten Celluloseethers vom Rest der Mischung und
   c) Gewinnen oder Entsorgen des veresterten Celluloseethers, der in der wässrigen Flüssigkeit gelöst ist.

2. Ein Verfahren zur Herstellung eines veresterten Celluloseethers, welcher Gruppen der Formel -C(O)-R-COOH enthält, worin R eine divalente Kohlenwasserstoffgruppe ist, wobei das Verfahren die Schritte umfasst

   a) Umsetzen eines Celluloseethers mit einem Dicarbonsäureanhydrid oder mit einer Kombination aus einem Dicarbonsäureanhydrid und einem aliphatischen Monocarbonsäureanhydrid in Gegenwart einer aliphatischen Carbonsäure, um ein Reaktionsproduktgemisch enthaltend eine veresterten Celluloseether, welcher Gruppen der Formel -C(O)-R-COOH enthält, herzustellen, Ausfällen des veresterten Celluloseethers von dem Reaktionsproduktgemisch, Mischen des ausgefällten veresterten Celluloseethers mit einer wässrigen Flüssigkeit und Einstellen der Temperatur der resultierenden Mischung auf weniger als 10°C, um einen Teil des veresterten Celluloseethers in der wässrigen Flüssigkeit zu lösen,

b) Trennen des nichtgelösten Teils des veresterten Celluloseethers von dem Rest der Mischung, und

c) Gewinnen oder Entsorgen des veresterten Celluloseethers, welcher in der wässrigen Flüssigkeit gelöst ist.

3. Verfahren nach Anspruch 1 oder 2, wobei Schritt a) das Einstellen der Temperatur der resultierenden Mischung auf eine Temperatur von weniger als 8°C umfasst.

4. Verfahren nach Anspruch 1 oder 2, wobei Schritt a) das Einstellen der Temperatur der resultierenden Mischung auf eine Temperatur von weniger als 5°C umfasst.

5. Verfahren nach einem der Ansprüche 1 bis 4, wobei Schritt a) Mischen des veresterten Celluloseethers mit der wässrigen Flüssigkeit in einem Gewichtsverhältnis von 5 bis 100 Gewichtsteilen wässriger Flüssigkeit pro einem Gewichtsanteil veresterten Zelluloseether umfasst.

6. Verfahren nach einem der Ansprüche 1 bis 5, wobei in Schritt c) der veresterte Zelluloseether durch Erhitzen der wässrigen Flüssigkeit, welche gelösten veresterten Zelluloseether umfasst, auf eine Temperatur von mindestens 30°C oder durch Gefriertrocknen gewonnen wird.

7. Verfahren nach einem der Ansprüche 1 bis 6, wobei der veresterte Zelluloseether Gruppen der Formel -C(O)-CH2-CH2-COOH enthält.

8. Das Verfahren nach einem der Ansprüche 1 bis 7, wobei der veresterte Zelluloseether zusätzlich aliphatische monovalente Acylgruppen enthält.

9. Verfahren nach einem der Ansprüche 1 bis 8, wobei der veresterte Zelluloseether Hydroxypropylmethylcelluloseacetatsuccinat ist.

10. Verfahren nach einem der Ansprüche 1 bis 9, wobei ein veresterter Zelluloseether, der ein gewichtsmittleres Molekulargewicht $M_w$ von mindestens 80.000 Dalton und eine Polydispersität $M_w/M_n$ von nicht mehr als 2,6 hat, in Schritt b) enthalten wird.

11. Das Verfahren nach einem der Ansprüche 1 bis 10, wobei ein veresterter Zelluloseether, der ein gewichtsmittleres Molekulargewicht $M_w$ von bis zu 70.000 Dalton und eine Polydispersität $M_w/M_n$ von nicht mehr als 2,6 hat, in Schritt c) enthalten wird.

**Revendications**

1. Un procédé de fractionnement d'un éther de cellulose estérifié comprenant des groupes de la formule -C(O)-R-COOH, dans laquelle R est un groupe hydrocarboné divalent, lequel procédé comprend les étapes consistant à

a) mélanger de manière homogène l'éther de cellulose estérifié comprenant des groupes de la formule -C(O)-R-COOH avec un liquide aqueux et régler la température du mélange homogène résultant à moins de 10 °C afin de dissoudre une portion de l'éther de cellulose estérifié dans le liquide aqueux,

b) séparer la portion non dissoute de l'éther de cellulose estérifié du reste du mélange homogène, et

c) récupérer ou éliminer l'éther de cellulose estérifié qui est dissous dans le liquide aqueux.

2. Un procédé de préparation d'un éther de cellulose estérifié comprenant des groupes de la formule -C(O)-R-COOH, dans laquelle R est un groupe hydrocarboné divalent, lequel procédé comprend les étapes consistant à

a) faire réagir un éther de cellulose avec un anhydride d'acide dicarboxylique ou avec une combinaison d'un anhydride d'acide dicarboxylique et d'un anhydride d'acide monocarboxylique aliphatique en présence d'un acide carboxylique aliphatique afin de produire un mélange de produits de réaction comprenant un éther de cellulose estérifié comprenant des groupes de la formule -C(O)-R-COOH, faire précipiter l'éther de cellulose estérifié à partir du mélange de produits de réaction, mélanger de manière homogène l'éther de cellulose estérifié ayant précipité avec un liquide aqueux et régler la température du mélange homogène résultant à moins de 10 °C afin de dissoudre une portion de l'éther de cellulose estérifié dans le liquide aqueux,

b) séparer la portion non dissoute de l'éther de cellulose estérifié du reste du mélange homogène, et

c) récupérer ou éliminer l'éther de cellulose estérifié qui est dissous dans le liquide aqueux.

3. Le procédé de la revendication 1 ou de la revendication 2 dans lequel l'étape a) comprend le réglage de la température du mélange homogène résultant à une température inférieure à 8 °C.

4. Le procédé de la revendication 1 ou de la revendication 2 dans lequel l'étape a) comprend le réglage de la température du mélange homogène résultant à une température inférieure à 5 °C.

5. Le procédé de l'une quelconque des revendications 1 à 4 dans lequel l'étape a) comprend le mélange homogène de l'éther de cellulose estérifié avec le liquide aqueux à un rapport en poids allant de 5 à 100 parties en poids de liquide aqueux pour une partie en poids d'éther de cellulose estérifié.

6. Le procédé de l'une quelconque des revendications 1 à 5 dans lequel, à l'étape c), l'éther de cellulose estérifié est récupéré par chauffage du liquide aqueux comprenant de l'éther de cellulose estérifié dissous jusqu'à une température d'au moins 30 °C ou par lyophilisation.

7. Le procédé de l'une quelconque des revendications 1 à 6 dans lequel l'éther de cellulose estérifié comprend des groupes de la formule - C(O) - CH2 - CH2 - COOH.

8. Le procédé de l'une quelconque des revendications 1 à 7 dans lequel l'éther de cellulose estérifié comprend en outre des groupes acyle monovalents aliphatiques.

9. Le procédé de l'une quelconque des revendications 1 à 8 dans lequel l'éther de cellulose estérifié est l'acétate succinate d'hydroxypropylméthylcellulose.

10. Le procédé de l'une quelconque des revendications 1 à 9 dans lequel un éther de cellulose estérifié ayant une masse moléculaire moyenne en poids Mw d'au moins 80 000 Dalton et une Polydispersité Mw/Mn de pas plus de 2,6 est obtenu à l'étape b).

11. Le procédé de l'une quelconque des revendications 1 à 10 dans lequel un éther de cellulose estérifié ayant une masse moléculaire moyenne en poids Mw allant jusqu'à 70 000 Dalton et une Polydispersité Mw/Mn de pas plus de 2,6 est obtenu à l'étape c).

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4365060 A
- US 4226981 A
- WO 2005115330 A
- WO 2014031447 A
- US 3435027 A
- WO 2013148154 A
- EP 0219426 A

**Non-patent literature cited in the description**

- **MCGINITY ; JAMES W.** Aqueous Polymeric Coatings for Pharmaceutical Dosage Forms. M. Dekker, 1989, 105-113
- *Hypromellose Acetate Succinate, United States Pharmacopia and National Formulary, NF,* vol. 29, 1548-1550
- United States Pharmacopeia and National Formulary, USP. *Hypromellose,* vol. 35, 3467-3469
- *Hypromellose Acetate Succinate, United States Pharmacopeia and National Formulary, NF,* vol. 29, 1548-1550
- *Ubbelohde viscosity measurement according to DIN,* January 1999, 51562-1
- *Journal of Pharmaceutical and Biomedical Analysis,* 2011, vol. 56, 743
- *Journal of Pharmaceutical and Biomedical Analysis,* 2011, vol. 56, 743-747
- **CHEN, R. et al.** *Journal of Pharmaceutical and Biomedical Analysis,* 2011, vol. 56, 743-748
- *MOLECULAR PHARMACEUTICS,* vol. 5 (6), 1003-1019